# EUROPEAN PATENT APPLICATION

(11) **EP 4 215 267 A1**
(43) Date of publication of application: **26.07.2023**
(21) Application number: 22305074.1
(22) Date of filing: 25.01.2022
(51) Int. Cl.: B01J 23/04, B01J 27/232, B01J 27/10, B01J 37/08, B01J 37/36, C07C 2/00, B01J 19/12, C11B 9/00

(54) **NOVEL COMPOSITIONS FOR THE SUSTAINABLE CATALYSIS OF ORGANIC SYNTHESIS REACTIONS**

(71) Applicant: Centre national de la recherche scientifique, 75016 Paris (FR); Université de Montpellier, 34090 Montpellier (FR)
(72) Inventor: GRISON, Claude, 34170 CASTELNAU LE LEZ FRANCE (FR); LOCK TOY KI, Yvette, 34980 COMBAILLAUX (FR)
(74) Representative: Lavoix

(57) **Abstract**

The present invention relates to a comprising K₂CO₃, KCI, and optionally K₂SO₄ and/or KHCO₃, wherein the weight content of potassium is between 9.0 and 60.0 % relative to the total weight of the composition, preferably between 10.0 and 50.0%, more preferably between 10.0 and 40.0%, advantageously between 20.0 and 40.0%.

## Description

The present invention concerns compositions comprising specific potassium salts and specific amount of potassium.

The invention also concerns methods for the preparation of these compositions.

The invention also concerns the use of these compositions as catalyst for chemical synthesis reactions.

### STATE OF THE ART

Many basic compounds are known as reactants or catalysts for organic synthesis reactions. However, they are usually obtained by conventional chemical processes, which often have a high environmental impact. Also, the conditions under which these organic reactions are implemented require the use of non-biosourced reagents, organic solvents, high temperature conditions that increase the energy balance, purification steps, and form large amounts of waste.

Nevertheless, the society is increasingly demanding products obtained by processes with a reduced ecological impact. In particular, organic and ethical sourcing of ingredients, eco-design and eco-friendly processes, waste management and green chemistry are becoming priorities.

In this context, basic catalysts derived from natural material rich in calcium (comprising more than 50000 ppm of calcium), in particular from plants rich in calcium oxalate and from natural materials rich in calcium carbonates, were developed and are described for example in WO 2015/036714. To obtain such basic catalysts, these natural materials undergo several thermal and basic/acid treatments. Around 550°C, calcium oxalates comprised in the plants are transformed into calcium carbonates, and around 800°C, calcium carbonates are transformed into calcium oxides. Through a hydration reaction, calcium oxides are transformed into calcium hydroxides. Calcium hydroxides can also be obtained from calcium carbonate by a chloro-acidic treatment, giving calcium chloride, followed by an alkaline hydrolysis. These catalysts were involved in several organic reactions.

However, these processes to obtain the final catalyst involve several thermal and basic/acid treatments that increase their environmental balance. Also, some organic reactions, very difficult to implement following classical synthesis techniques, cannot be carried out even with catalysts described in WO 2015/036714.

### AIMS OF THE INVENTION

One aim of the present invention is to solve the technical problem of providing a composition displaying good basic catalytic performances.

Another aim of the present invention is to provide a composition obtain through a process with a reduced environmental impact.

Another aim of the present invention is to provide methods for implementing organic syntheses that are difficult to implement following known synthesis techniques and/or in the presence of catalysts of prior art.

Another aim of the present invention is to provide methods for implementing organic syntheses with a reduced environmental impact.

### DETAILLED DESCRIPTION OF THE INVENTION

The present invention relates to a composition comprising K₂CO₃, KCI, and optionally K₂SO₄ and/or KHCO₃, wherein the weight content of potassium is between 9.0 and 60.0 % relative to the total weight of the composition, preferably between 10.0 and 50.0%, more preferably between 10.0 and 40.0%, advantageously between 20.0 and 40.0%.

KHCO₃ and K₂CO₃ can be under anhydrous form or hydrated form. KHCO₃ and K₂CO₃ can also be present as co-crystalline species, for example K₂H(CO₃)_{1,5}(H₂O)_{0,75}.

The inventors have discovered that starting from plants wherein the above-ground parts comprise a very high amount potassium, they were able to obtain a composition according to the invention. Unexpectedly, these compositions were found to be very effective as catalysts in several organic reactions.

Preferably, the composition according to the invention is a polymetallic composition.

According to an embodiment, the composition further comprises sodium, calcium and magnesium.

According to an embodiment, the composition further comprises iron and aluminum.

Preferably, the composition comprises between 0.0001 and 0.30% by weight of sodium, preferably between 0.0001 and 0.25% by weight, relative to the total weight of the composition.

Preferably, the composition comprises between 0.1 and 20.0% by weight of calcium, preferably between 1.0 and 20.0% relative to the total weight of the composition. According to an embodiment, the composition comprises between 0.1 and 5.0% by weight of calcium, preferably between 0.5% and 4.0% by weight, more preferably between 1.0 and 3.0% by weight, relative to the total weight of the composition. According to another embodiment, the composition comprises between 5.0 and 20.0% by weight of calcium relative to the total weight of the composition.

Preferably, the composition comprises between 0.0001 and 10.0% by weight of magnesium, relative to the total weight of the composition. According to an embodiment, the composition comprises between 2.5 and 10.0% by weight of magnesium, preferably between 3.0 and 10.0% by weight relative to the total weight of the composition. According to another embodiment, the composition comprises between 0.0001 and 3.0% by weight of magnesium, preferably between 0.1 and 3.0% by weight, relative to the total weight of the composition.

Preferably, the composition comprises between 0.0001 and 1.0% by weight of iron, preferably between 0.001 and 0.8% by weight, more preferably between 0.01 and 0.6% by weight, relative to the total weight of the composition.

Preferably, the composition comprises between 0.0001 and 1.0% by weight of aluminum, relative to the total weight of the composition. According to an embodiment, the composition comprises between 0.05 and 0.8% by weight of aluminum, relative to the total weight of the composition. According to another embodiment, the composition comprises between 0.0001 and 0.1% by weight of aluminum, preferably between 0.01 and 0.08%, relative to the total weight of the composition.

According to an embodiment, the composition further comprises manganese.

Preferably, the composition comprises between 0.0001 and 0.30% by weight of manganese, preferably between 0.005 and 0.30% by weight, more preferably between 0.01 and 0.30% by weight, advantageously between 0.01 and 0.20% by weight, relative to the total weight of the composition. According to an embodiment, the composition comprises between 0.0001 and 0.1% by weight of manganese, preferably between 0.005 and 0.05% by weight, relative to the total weight of the composition.

According to an embodiment, the composition further comprises zinc.

Preferably, the composition comprises between 0.0001 and 0.4% by weight of zinc, preferably between 0.005 and 0.3%, more preferably between 0.01 and 0.2%, relative to the total weight of the composition.

The content of each metal has been determined by microwave plasma atomic emission spectrometry (MP-AES) directly on the composition.

Analyses are performed using the metal analysis of total dissolved composition in water. For this purpose, the composition is digested in 10 mL of reversed *aqua regia* (1:2 hydrochloric acid (37%): nitric acid (65%)) under a microwave-assisted digestion (Multiwave-Go Anton Paar) with the following program: 20 to 165 °C in 20 min and then 10 min isothermal at 165 °C. Samples were filtered and then diluted to 0.4 mg.L⁻¹ in 1% aqueous nitric acid. Mineral composition is determined by using a microwave plasma-atomic emission spectroscopy (MP-AES) 4200 (Agilent Technologies) equipped with a concentric nebulizer and a double-pass cyclonic spray chamber. The pump speed during analysis was kept at 10 rpm and the sample introduction tube diameter was 0.89 mm. The analytical cycle consists of 30 s rinsing with aq. 1% nitric acid followed by 25 s of sample uptake (pump speed 40 rpm) and then 20 s of equilibration before the reading at preselected integration times (pump speed 10 rpm). The integration time is set to 3 s for all elements. Unless otherwise stated, the automatic background correction mode available in the software was used. All analysis results are performed in triplicate.

According to an embodiment, the composition is substantially devoid of calcium hydroxide Ca(OH)₂.

More preferably, the composition is completely devoid of calcium hydroxide Ca(OH)₂.

According to an embodiment, the composition of the invention is substantially devoid of calcium oxide CaO. More preferably, the composition is completely devoid of calcium oxide CaO.

Preferably, the composition of the invention is substantially devoid of calcium oxide CaO and of calcium hydroxide Ca(OH)₂. More preferably, the composition is completely devoid of calcium oxide CaO and of calcium hydroxide Ca(OH)₂.

According to an embodiment, the composition of the invention is substantially devoid of calcium hydroxide Ca(OH)₂, potassium hydroxide KOH and magnesium hydroxide Mg(OH)₂. Preferably, the composition is completely devoid of calcium hydroxide Ca(OH)₂, potassium hydroxide KOH and magnesium hydroxide Mg(OH)₂. Preferably, the composition of the invention is substantially devoid of calcium oxide CaO, calcium hydroxide Ca(OH)₂, potassium hydroxide KOH and magnesium hydroxide Mg(OH)₂. More preferably, the composition is completely devoid of calcium oxide CaO, calcium hydroxide Ca(OH)₂, potassium hydroxide KOH and magnesium hydroxide Mg(OH)₂.

According to an embodiment, the composition of the invention is substantially devoid of metallic hydroxide. Preferably, the composition is completely devoid of metallic hydroxide. Preferably, the composition of the invention is substantially devoid of metallic hydroxide and of calcium oxide CaO. More preferably, the composition is completely devoid of metallic hydroxide and of calcium oxide CaO.

By "substantially devoid" of calcium hydroxide, potassium hydroxide, magnesium hydroxide, calcium oxide or metallic hydroxide, it is meant less than 0.1% by weight, preferably less than 0.01% by weight, more preferably less than 0.001% by weight relative to the total weight of the composition of calcium hydroxide, potassium hydroxide, magnesium hydroxide, calcium oxide or metallic hydroxide.

A metallic hydroxide is a compound of formula M^{x+}(OH)ₓ, x being an integer from 1 to 3, and M being a metal selected from Ca, Mg, Zn, Mn, K and Fe.

Despite the absence of Ca(OH)₂, these compositions were found to be very effective as catalysts in several organic reactions. This result was not expected because the compositions of prior art, in particular of WO 2015/036714, are mainly composed of calcium hydroxide, which is the active species in the organic synthesis reactions carried out.

According to an embodiment, the composition is substantially devoid of metals selected from the group of platinoids and in particular Pt, Pd or Rh, and/or of rare earths, in particular Ce, Eu, and Yb; and/or of metalloids, in particular B, Ge, As, Sb and Te.

According to an embodiment, the composition is substantially devoid of transitions metals selected from the group consisting of Sc, Y, Ti, Zr, Hf, V, Nb, Ta, Cr, Mo, W, Tc, Re, Ru, Os, Co, Rh, Ir, Ni, Pd, Pt, Cu, Ag, Au and Cd.

By "substantially devoid of metals selected from the group of ...", it is meant that each metal is present in a quantity of less than 0.6 % by weight, preferably less than 0.4 % by weight, more preferably 0.2 % by weight relative to the total weight of the composition.

The inventors have observed it is preferable that the amount of these metals is as low as possible to obtain better catalytic performances of the composition according to the invention. Consequently, the composition according to the invention is completely different from the compositions described in WO2018178374. In WO2018178374, compositions are characterized by a high amount of metals selected from the group of platinoids (Pd, Pt, Rh), rare earths (Ce, Eu, Yb) or from the group comprising Zn, Mn, Ni, Cu, Fe, Al, Ca, Mg, or from the group comprising As, Sb, Cr, Cd, Pb, Ni, Co, as these composition were obtained by contacting a material of plant origin from a dead plant with an effluent comprising at least one of these metals. Consequently, the resulting compositions comprise a high amount of said metals, which is preferably not the case for the composition of the present invention.

The invention also relates to a method for the preparation of a composition according to the invention, comprising the following steps:
- selecting a plant, wherein the above-ground parts of said plant comprising between 10.0 and 40.0 % by weight of potassium,
- performing a heat treatment under air and at a temperature between 450°C and 650°C of the above-ground parts of said plant, and
- obtaining the composition.

By above-ground parts of a plant, it is meant the aerial parts of a plant, i.e. every part of the plant growing in a soil that is visible. The aerial part of plants are all the structures of a plant that are above ground, including the stems, leaves, petioles, flowers, fruits and seeds.

Preferably, the above-ground parts of the plant are the leaves and/or the stems and/or the ramifications and/or the petioles of the plant.

According to an embodiment, the above-ground parts of the plant are a mixture of two, preferably three parts of the plant among the list consisting of the leaves, the stems, the ramifications and the petioles of the plant. More preferably, the above-ground parts of the plant are a mixture of the leaves, the stems, the ramifications and the petioles of the plant.

To obtain a composition according to the invention, the suitable plants are selected by measuring the percentage by weight of K of the above-ground parts of said plant. The weight percentage of K of the above-ground parts of a plant is determined by MP-AES after a heat treatment under air at 550°C during 4 hours. A plant is suitable to obtain a composition according to the invention if its above-ground parts comprise between 10.0 and 40.0 % by weight of potassium.

Preferably, the heat treatment of the method for preparation of a composition according to the invention is carried out under air. The above-ground parts of said plant

Preferably, the heat treatment is performed during a time period comprised between 3 hours and 5 hours, advantageously around 4 hours.

Advantageously, the heat treatment is performed at a temperature between 400°C and 700°C, preferably between 450 °C and 650 °C, more preferably between 530 and 560 °C, even more preferably around 550°C.

Surprisingly, the inventors discovered that only a single thermal treatment of said above-ground parts allows obtaining the composition of the invention. In particular, no further heat treatment at higher temperature than the temperature of the heat treatment of the method for the preparation of a composition according to the invention is required.

According to an embodiment, the method for the preparation of a composition according to the invention is devoid of a step of acid treatment and/or is devoid of a basic treatment and/or is devoid of a purification step, for example by an ion exchange resin, a liquid-liquid extraction, a selective precipitation or a liquid/solid extraction and/or is devoid of an activation step.

According to an embodiment, in the method for the preparation of a composition according to the invention, the plant belongs to the genus Fallopia, Salix or Arundo.

The genus "Fallopia" is also known as "Reynoutria".

According to an embodiment, in the method for the preparation of a composition according to the invention, the plant is *Fallopia japonica, Salix alba* or *Arundo donax.*

Preferably, the heat treatment is performed on the leaves or the stems of *Fallopia japonica,* or on the leaves of *Salix alba,* or on the stems of *Arundo donax.*

The invention also relates to a composition obtainable from the method described above.

The composition according to the invention can be advantageously used as catalyst for chemical synthesis reaction.

The invention also relates to a method of carrying out a chemical synthesis reaction comprising a step of contacting the composition according to the invention with the reactant(s) of a chemical synthesis reaction, said chemical synthesis reaction being selected from the group consisting of 1,4-nucleophilic additions, aldolization/crotonisation reactions and similar reactions, transfunctionalisation reactions and oxidative hydroxylation reactions.

According to an embodiment, the method of carrying out a chemical synthesis reaction comprises:
- a step of dissolving the crude mixture obtained at the end of the step of contacting the composition with the reactant(s) in an organic solvent, preferably ethyl acetate, to obtain an organic solution with insoluble compound, followed by
- a filtration step to separate insoluble compounds from the organic solution, and
- an evaporation step of the organic solvent of the organic solution to obtain organic product(s).

Optionally, if the organic product(s) is a mixture of at least two organic products, the method of carrying out a chemical synthesis reaction further comprises a step of purification of the organic product(s), for example by distillation, preferably under vacuum.

According to an embodiment, the method of carrying out a chemical synthesis reaction comprises:
- a filtration step to separate insoluble compounds from the crude mixture obtained at the end of the step of contacting the composition with the reactant(s), followed by
- a distillation, preferably under vacuum, to remove excess reactant(s).

Excess reactant(s) can be recycle and reuse.

According to an embodiment, the method of carrying out a chemical synthesis reaction further comprises a step of thermal treatment of the insoluble compounds separated from the organic products, said thermal treatment being carried out at a temperature between 400 °C and 700°C, preferably between 450 °C and 650 °C, more preferably between 530 and 560 °C, to give a reactivated composition. Preferably, said thermal treatment is carried out during from 2 hours to 6 hours.

This step of thermal treatment of the insoluble compounds allows the recycling of the composition. The reactivated composition can be reused in another method of carrying out a chemical synthesis reaction.

According to an embodiment, the method of carrying out a chemical synthesis reaction according of the invention is performed in the absence of organic solvent, preferably in the absence of any solvent.

By "in the absence of organic solvent", or "in the absence of solvent", it is meant that the chemical transformation of the reactant(s) does not occur in an organic solvent, or in any other solvent, respectively. A solvent is not a reactant of the chemical synthesis reaction. This does not exclude the use of an organic solvent or of a solvent for the treatment of the crude material obtained after the chemical transformation of the reactant(s).

According to an embodiment, the step of contacting the composition according to the invention with the reactant(s) during the method of carrying out a chemical synthesis reaction is performed under an activation method.

Preferably, the step of contacting the composition according to the invention with the reactant(s) is performed by mecanosynthesis or under microwaves.

A mechanosynthesis is a chemical synthesis using mechanical energy to promote the chemical reaction. A mechanosynthesis usually starts from solids, usually without any solvent. The source of mechanical energy used to force molecules to react is classically obtained by grinding a physical mixture of two (or more) different partners in a jar of a ball mill. Such an equipment can be a Planetary Ball Mill Machine referenced TMAX-XQM-12 or RETSCH PM100.

Preferably, the mechanosynthesis is performed at a temperature comprised between 25°C and 80°C, preferably between 25°C and 60°C, more preferably between 25°C and 40°C.

Preferably, the mechanosynthesis is performed in a ball miller comprising balls having a diameter comprised between 3 millimeters and 35 millimeters. The rotation speed is preferably comprised between 250 rpm and 750 rpm, more preferably around 500 rpm. Preferably a pause in the rotation of the balls is done after each hour of reaction. Preferably the pause duration is comprised between 5 minutes and 30 minutes.

The use of a mechanosynthesis step allows obtaining very high conversion rate and final yield. The use of a mechanosynthesis step allows reducing the amount of excess reactant.

According to an embodiment, the synthesis under microwaves is performed under a pressure comprised between 1 and 55 bars.

Example of an equipment for carrying a synthesis under microwaves is Microwave Accelerated Reaction System for Synthesis, Model MARS 6, 240V/50Hz.

The combination of the composition of the invention with mechanosynthesis or with microwave activation allows performing reactions under milder conditions than those of conventional processes. Thus, the method of carrying out a chemical synthesis reaction according to the invention allows implementing organic syntheses that are difficult to implement following known synthesis techniques and/or in the presence of catalysts of prior art, in particular synthesis involving reactant(s) that tend to degrade under the conditions of said known synthesis techniques. Thus, the method of carrying out a chemical synthesis reaction according to the invention gives access to new products that are not accessible or hardly accessible following conventional processes.

According to an embodiment, the step of contacting the composition with the reactant(s) of the 1,4-nucleophilic additions is carried out in the absence of organic solvent, preferably in the absence of any solvent, more preferably by mechanosynthesis or under microwaves, advantageously by mechanosynthesis.

By "1,4-nucleophilic additions", it is meant the addition reactions of a nucleophile on the β position of an α,β-unsaturated carbonyl compounds. Examples of 1,4-nucleophilic additions are:
- the reaction of conjugated carbonyls with secondary amines to form 3-aminocarbonyls (3-ketoamines),
- the reaction of conjugated carbonyls with hydrogen cyanide to 1,4-keto-nitriles,
- the Michael addition,
- the Stork enamine reaction involving the conjugate addition of enamines to conjugated carbonyls.

The 1,4-nucleophilic addition can be intramolecular or intermolecular.

Preferably, the 1,4-nucleophilic addition is a Michael type addition.

Preferably, the 1,4-nucleophilic addition comprises the reaction between a compound bearing a nucleophilic carbon and a compound bearing an α,β-unsaturated carbonyl function, or the self-reaction of a compound bearing both a nucleophilic carbon and an α,β-unsaturated carbonyl function.

Preferably, the 1,4-nucleophilic addition is the reaction between a compound bearing an α,β-unsaturated carbonyl function of formula [1] and a compound bearing a nucleophilic carbon of formula [2]. 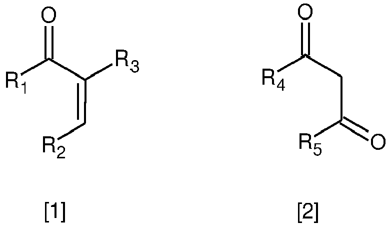
wherein R₁ and R₂ are independently selected in group consisting in H, a linear, cyclic or branched, saturated or unsaturated, optionally substituted, hydrocarbon group comprising from 1 to 8 carbon atoms, or form a ring comprising from 5 to 7 members, or form an aryl ring,
wherein R₃ is selected in the group consisting in H, and a linear, cyclic or branched, saturated or unsaturated, optionally substituted, hydrocarbon group comprising from 1 to 10 carbon atoms, preferably is H or a saturated linear hydrocarbon group comprising from 1 to 10 carbon atoms, more preferably is H or C₅H₁₁, and
wherein R₄ and R₅ are independently selected in the group consisting in H, a linear, cyclic or branched, saturated or unsaturated, optionally substituted, hydrocarbon group comprising from 1 to 8 carbon atoms and a group of formula -O-A, A representing a linear, cyclic or branched, saturated or unsaturated, optionally substituted, hydrocarbon group comprising from 1 to 6 carbon atoms, or form a ring comprising from 5 to 7 members, preferably are independently selected in group consisting in -OMe and Me, or form a saturated 6-member ring.

Advantageously, the compound of formula [1] is 2-cyclopenten-1-one or a derivative of 2-cyclopenten-1-one wherein at least one of the carbon of the cycle has a C1-C8 saturated alkyl group, advantageously is 2-pentyl-2-cyclopenten-1-one.

Advantageously, the compound of formula [2] is dimethyl malonate.

According to a specific embodiment, the 1,4-nucleophilic addition is followed by a intramolecular aldolic condensation, followed by an intramolecular crotonisation, followed by an intramolecular decarboxylation reaction. Preferably, R₃ = H, R₄ = Me and R₅ = OMe. More preferably, R₃ = H, R₄ = Me, R₅ = OMe and R₂ = H. Even more preferably, R₃ = H, R₄ = Me, R₅ = OMe, R₂ = H and R₁ = H or Me.

According to an embodiment, the 1,4-nucleophilic additions are carried out in the presence of an amount of composition corresponding to between 0.01 equivalents and 5 equivalents of K relative to the limiting reactant, preferably between 0.05 equivalents and 4 equivalents, more preferably between 0.1 equivalent and 3 equivalents, advantageously between 0.1 equivalent and 2.5 equivalents.

As the percentage by weight of K is determinable by MP-AES following the method described above, the amount of composition to be used is easily determined by the skilled person.

Preferably, the 1,4-nucleophilic additions are carried out in the presence of one to ten equivalents of the compound bearing a nucleophilic carbon (compound of formula [2]) with respect to the amount of the compound bearing an α,β-unsaturated carbonyl function (compound of formula [1]).

According to an embodiment, in the method of carrying out a chemical synthesis reaction according to the invention, the chemical synthesis reaction comprises a 1,4-nucleophilic addition, followed by an aldolization/crotonisation reaction, followed by a decarboxylation reaction.

By "aldolization/crotonisation reactions and similar reactions", it is meant all the condensation reactions involving the addition of a carbon bearing an activated hydrogen (i.e. an acid hydrogen) on a carbon from a C=O or a C=N group. Examples of aldolization/crotonisation and similar reactions are: aldolic condensation of aldehydes, cetolic condensation of ketones, cross-condensation between aldehyde, ketone and esters, the Knoevenagel reaction, the Dieckmann reaction.

The aldolization/crotonisation reactions and similar reactions can be intramolecular or intermolecular reactions.

Preferably, the aldolization/crotonisation reactions and similar reactions comprises the condensation reaction between a compound bearing an enolizable aldehyde function or an enolizable ketone function or an enolizable ester function and a compound bearing an aldehyde function, a ketone function or an ester function. Alternatively, the aldolization/crotonisation reactions and similar reactions comprises the self-reaction of a compound bearing both an enolizable aldehyde function or an enolizable ketone function or an enolizable ester function and an aldehyde function, a ketone function or an ester function.

Preferably, the aldolization/crotonisation and similar reaction is the reaction between a compound of formula [9] and a compound of formula [10]. 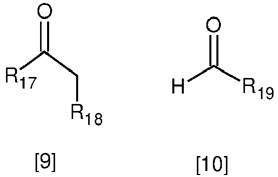
wherein R₁₇ and R₁₈ are independently a linear, cyclic or branched, saturated or unsaturated, optionally substituted, hydrocarbon group comprising from 1 to 8 carbon atoms, or form a ring comprising from 5 to 7 members, preferably form a saturated 5-member ring,
wherein R₁₉ is a linear, cyclic or branched, saturated or unsaturated, optionally substituted, hydrocarbon group comprising from 1 to 10 carbon atoms, preferably a saturated linear hydrocarbon group comprising from 1 to 10 carbon atoms, more preferably is C₅H₁₁.

Advantageously, the aldolization/crotonisation and similar reaction is the aldolisation followed by the crotonisation of cyclopentanone and pentanal

The aldolization/crotonisation and similar reaction can also be the reaction between a compound of formula [13] and a compound of formula [5] 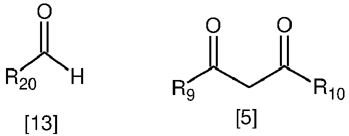 wherein R₂₀ is selected in the group consisting of a phenyl group and a linear, cyclic or branched, saturated or unsaturated, optionally substituted, hydrocarbon group comprising from 1 to 8 carbon atoms, wherein R₉ and R₁₀ are independently selected in the group consisting in H, a linear, cyclic or branched, saturated or unsaturated, optionally substituted, hydrocarbon group comprising from 1 to 8 carbon atoms and a group of formula -O-A, A representing a linear, cyclic or branched, saturated or unsaturated, optionally substituted, hydrocarbon group comprising from 1 to 6 carbon atoms, or form a ring comprising from 5 to 7 members.

According to an embodiment, the reaction between a compound of formula [13] and a compound of formula [5] is followed by a decarboxylation reaction. Preferably, R₁₀ = OMe. More preferably, R₂₀ = Ph, R₉ = Me and R₁₀ = OMe.

The aldolization/crotonisation and similar reaction can also be the intramolecular reaction of a compound of formula [32] 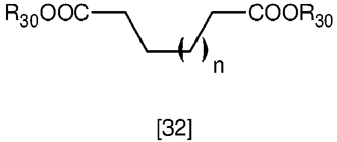 wherein n is an integer equal to 1, 2 or 3, preferably equal to 1, and R₃₀ is a linear or branched, saturated or unsaturated hydrocarbon group comprising from 1 to 8 carbon atoms, preferably a saturated linear hydrocarbon group comprising from 1 to 8 carbon atoms, more preferably is CH₃.

Preferably, the aldolization/crotonisation reactions and similar reactions are carried out in the presence of an amount of composition corresponding to between 0.01 equivalents and 2.0 equivalents of K relative to the limiting reactant, preferably between 0.02 equivalents and 1.0 equivalents, more preferably between 0.05 equivalents and 0.5 equivalents.

A transfunctionnalization reaction is a reaction during which the substituent(s) of a chemical function are replaced by others substituent(s).

Preferably, transfunctionnalisations reactions are transesterification reactions and transcarbonatation reactions.

By "transesterification reactions", it is meant a reaction during which an organic group R' of an ester function -C(O)-OR' of a compound is exchanged with the organic group R" of an alcohol R"OH to give a compound comprising another ester function -C(O)-OR" and an alcohol R'OH.

The transesterification reaction is preferably a reaction between a compound comprising at least one OH function and a compound comprising at least an ester function. Preferably the compound comprising at least one OH function is selected in the group consisting of alcohol of formula R"OH wherein R" is a C1-C10 saturated or a C2-C10 unsaturated hydrocarbon group, a diol, for example ethylene glycol, propane-1,2-diol, butane-1,2-diol, butane-2,3-diol, propane-1,3-diol, butane-1,4-diol, pentane-1,5-diol, hexane-1,6-diol, octane-1,8-diol, butane-1,3-diol, pentane-1,2-diol, p-menthane-3,8-diol, 2-méthylpentane-2,4-diol, and polyols, for example glycerol, erythritol, sorbitol, mannitol or polyglycerol.

More preferably, the compound comprising at least one OH function is methanol or ethanol, advantageously ethanol.

Preferably, the compound comprising at least an ester function is chosen from the fatty esters, the diesters and the triglycerides.

Preferably, the fatty ester is a compound of formula [15] 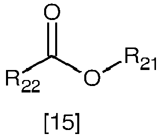 wherein R₂₁ is a hydrocarbon group comprising from 1 to 30 carbon atoms, preferably from 1 to 25 carbon atoms, linear or branched, saturated or comprising at least one unsaturation, optionally substituted for example by an OH group, and R₂₂ is selected in the group consisting of H, a hydrocarbon group comprising from 1 to 30 carbon atoms, preferably from 1 to 25 carbon atoms, linear or branched, saturated or comprising at least one unsaturation, and a linear, cyclic or branched, saturated or unsaturated, optionally substituted, hydrocarbon group comprising from 1 to 10 carbon atoms. More preferably, R₂₂ = H. Advantageously, the compound of formula [15] is geranyl formiate.

Preferably, the triglyceride is a compound of formula [19] 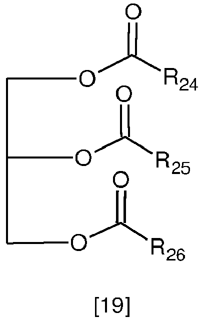 wherein R₂₄, R₂₅ and R₂₆ are independently a hydrocarbon group comprising from 1 to 30 carbon atoms, preferably from 1 to 25 carbon atoms, linear or branched, saturated or comprising at least one unsaturation.

Preferably, the compound comprising at least an ester function is a mixture of at least two triglycerides. More preferably, the compound comprising at least an ester function is chosen from vegetable oils. Even more preferably, the compound comprising at least an ester function is selected in the group consisting of olive oil, grapeseed oil, rapeseed oil, coconut oil, castor oil and sunflower oil.

Preferably, the molar ratio between the compound comprising at least an ester function and the compound comprising at least one OH function is inferior or equal to 65, more preferably inferior or equal to 40, advantageously between 4 and 10.

Preferably, the transesterification reactions are carried out under a pressure comprised between 1 bar and 4 bars, more preferably between 1 bar and 2.5 bars.

Preferably, the transesterification reactions are carried out at a temperature comprised between 25°C and 150°C, more preferably between 40°C and 120°C, advantageously between 60°C and 110°C.

Preferably, transesterification reactions are carried out during from 5 minutes to 2 hours, more preferably from 10 minutes to 1 hour, advantageously from 20 minutes to 40 minutes.

By "transcarbonatation reactions", it is meant a reaction during which organic groups R and R' of an carbonate function RO-C(O)-OR' of a compound are exchanged with the organic group R" and R'" of alcohols R"OH and R‴OH to give a compound comprising another carbonate function R‴O-C(O)-ORʺ and alcohols ROH and R'OH.

According to an embodiment, transesterification reactions further comprise a decantation step, followed by a filtration step. The insoluble part corresponds to the composition, that can be recycled and reused as described above. After filtration, the solution obtained can be evaporated to remove the excess of compound comprising at least one OH function. The excess compound can be recycled and reused in a further transesterification reaction.

Thanks to the use of the composition according to the invention as catalyst, transesterification reactions can be implemented even for substrates comprising unsaturations, as polyunsaturated triglycerides or ester of formic acids comprising at least one unsaturation, whereas conventional methods of transesterifications are not adapted for such substrates. This is due to the fact that the conditions are mild and does not require any hard reaction mixture treatment. More generally, the transesterification reactions according to the invention have the following advantages: 1/ heterogeneous catalysis (very rare) with recyclable catalyst; 2/ low catalytic load; 3/ no hydroxide and therefore no soap formation (common when KOH or NaOH is used); 4/ mild conditions (temperatures and pressures) and compatible with fragile substrates; 5/ fast reaction; 6/ even when the alcohol is EtOH, quantitative yields are obtained (very rare, reaction often limited to the used of methanol with triglycerides), thus the transesterification according to the invention allow obtaining 100% biobased fatty esters, which is impossible with MeOH, because unavailable in biobased form); 7/ 100% biobased process.

According to an embodiment, the transcarbonatation is a reaction between a compound comprising an alcohol function as describe above and a compound of formula [24] 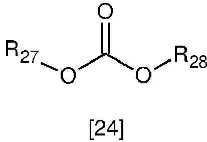 wherein R₂₇ and R₂₈ are independently a linear, cyclic or branched, saturated or unsaturated, optionally substituted, hydrocarbon group comprising from 1 to 8 carbon atoms. More preferably, R₂₇ and R₂₈ are a saturated linear hydrocarbon group comprising from 1 to 8 carbon atoms, advantageously is an ethyl group.

Preferably, the compound comprising an alcohol function is a polyol, advantageously is glycerol.

Preferably, the transcarbonatation reactions are carried out under atmospheric pressure, at a temperature comprised between 25°C and 200°C, more preferably between 80°C and 180°C, advantageously between 130°C and 160°C.

Preferably, transesterification reactions are carried out during from 10 minutes to 2 hours, more preferably from 30 minutes to 75 minutes, advantageously from 30 minutes to 1 hour.

Preferably, the transfunctionalisation reactions are carried out in the presence of an amount of composition corresponding to between 0.01 equivalents and 5.0 equivalents of K relative to the limiting reactant, preferably between 0.02 equivalents and 2.5 equivalents, more preferably between 0.05 equivalents and 1.5 equivalents.

According to an embodiment, the oxidative hydroxylation is the reaction between a compound comprising a phenone moiety or a benzaldehyde moiety, and an oxidant. Preferably, the oxidant is hydrogen peroxide.

Preferably, the phenone moiety or the benzaldehyde moiety comprises an OH group in ortho or para position of the ketone or aldehyde group.

Preferably, the oxidative hydroxylation is carried out in the presence of an amount of composition corresponding to between 0.01 equivalent and 0.3 equivalents of K relative to the compound comprising a phenone moiety or a benzaldehyde moiety, preferably between 0.05 equivalents and 0.1 equivalents.

Preferably, oxidative hydroxylation is carried out from 1 hour to 5 hours, more preferably from 2.5 hours to 3.5 hours.

Preferably, oxidative hydroxylation is carried out at a temperature comprised between 15°C and 30°C.

According to an embodiment, the aldolization/crotonisation reactions and similar reactions, and/or the transfunctionalisation reactions and/or the oxidative hydroxylation are carried out under microwaves.

The invention also relates to a method for the preparation of methyl dihydrojasmonate, comprising the following steps:
a) reacting cyclopentanone with pentanal in the presence of a composition according to the invention, to obtain a compound of formula [28] 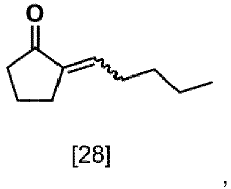
b) performing an isomerization of compound of formula [28], preferably under microwaves, to obtain compound of formula [29] 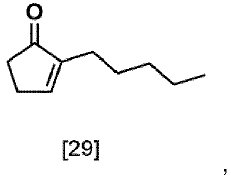
c) reacting compound of formula [29] with dimethyl malonate in the presence of a composition according to the invention, to obtain a compound of formula [30] 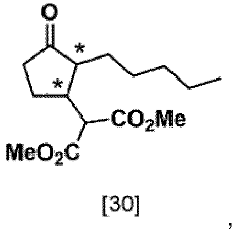
d) performing a decarboxylation of compound of formula [30], preferably under microwaves, to obtain methyl dihydrojasmonate.

Preferably, step a) is an aldolization/crotonisation reaction according to the present invention. Preferably, step a) is performed under microwaves. Preferably, step a) is performed in the absence of any organic solvent.

Preferably, step b) is performed at a temperature comprised between 100 °C and 200°C, more preferably between 130°C and 170°C. Preferably, step b) is performed during from 30 minutes to 3 hours, more preferably from 75 minutes to 2h30. Preferably, step b) is performed in the presence of glycerol.

Preferably, step c) is a 1,4-nucleophilic addition reaction according to the present invention. Preferably, step c) is performed in the absence of organic solvent, more preferably by mechanosynthesis.

Preferably, step d) is performed at a temperature comprised between 180 °C and 300 °C, more preferably between 230°C and 270°C. Preferably, step b) is performed during from 15 minutes to 2 hours, more preferably from 45 minutes to 75 minutes. Preferably, step b) is performed in the presence of succinic acid.

At the end of step d), methyl dihydrojasmonate is obtained as a mixture of cis isomers and trans isomers.

The two cis isomers of methyl dihydrojamonate are depicted below:

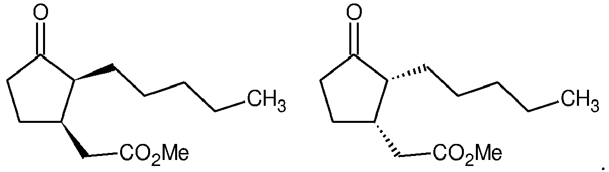

The two trans isomers of methyl dihydrojamonate are depicted below:

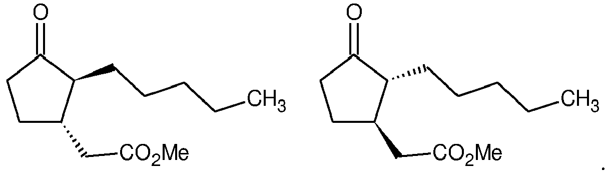

According to the invention, the amount of cis isomers represents the amount of the two cis-isomers relatively to the amount of the two cis isomers and the two trans isomers.

According to the invention, the amount of trans isomers represents the amount of the two trans-isomers relatively to the amount of the two cis isomers and the two trans isomers.

Preferably, the methyl dihydrojamonate obtained at the end of step d) comprises at least 10 molar % of cis isomers relative to the total molar quantity of the cis and trans isomers, preferably at least 15 molar %, more preferably at least 19 molar %, advantageously from 19 molar% to 20 molar %.

The method for the preparation of methyl dihydrojasmonate according to the invention has the following advantages:
- a majority of catalysts, reagents and substrates are biosourced,
- it does not comprise the use of non-biobased solvents,
- it limits the reaction treatments, thus reduces the formation of waste,
- each step involves modern green chemistry techniques, to control the stereoselectivity of the reaction and to shorten the reaction times,
- it allows reducing the number of steps: the transformation of compound 4 into methyl dihydrojasmonate is done in one step, (without toxic solvent like DMSO) and avoids the sequence saponification/protonation/decarboxylation/esterification usually performed,
- it avoids the use of corrosive and aggressive reagents during step b), as HCI (used in the patented process by Firmenich) or commonly used catalysts based on Pd or Rh are replaced by glycerol, a cheap and biosourced co-product, and
- it controls the stereochemistry during step c), because the conditions developed allow to obtain methyl dihydrojasmonate comprising at least 10% of cis isomers.

The present invention also relates to the use of a composition according to the invention, as a catalyst of a chemical synthesis reactions.

Preferably, the chemical synthesis reactions are selected from the group consisting of 1,4-nucleophilic additions, aldolization/crotonisation and similar reactions, transesterification reactions and oxidative hydroxylation reactions.

The invention will be better understood from reading the following non-limiting examples.

### EXAMPLES

### Example 1: Mineral compositions of some plant species

The mineral composition of parts of several plants is determined by MP-AES after a heat treatment under air flow at 550°C during 4 hours. MP AES analyses were performed using the metal analysis of total dissolved solutes in water. The samples were digested in 10 mL of reversed aqua regia (1:2 hydrochloric acid (37%): nitric acid (65%)) under a microwave-assisted digestion (Multiwave-Go Anton Paar) with the following program: 20 to 165 °C in 20 min and then 10 min isothermal at 165 °C. Samples were filtered and then diluted to 0.4 mg.L-1 in 1% aqueous nitric acid. Mineral compositions were determined by using a microwave plasma-atomic emission spectroscopy (MP-AES) 4200 (Agilent Technologies) equipped with a concentric nebulizer and a double-pass cyclonic spray chamber. The pump speed during analysis was kept at 10 rpm and the sample introduction tube diameter was 0.89 mm. The analytical cycle consisted of 30 s rinsing with aq. 1% nitric acid followed by 25 s of sample uptake (pump speed 40 rpm) and then 20 s of equilibration before the reading at preselected integration times (pump speed 10 rpm). The integration time was set to 3 s for all elements. Unless otherwise stated, the automatic background correction mode available in the software was used. All analysis results were performed in triplicate.

The following results were obtained. Content in each metal of each sample is designated as a weight percentage relative to the weight of the composition, more or less a percentage of standard deviation on the indicated value.

**Table 1**

| Plant: *Fallopia japonica* (EcoKOx-Fj-(X), X being from 1 to 13) | | | | | | | | | | | |
|---|---|---|---|---|---|---|---|---|---|---|---|
| **Harvesting site** | **N°** | **Harvesting date** | **Part of the plant** | **Al** | **Ca** | **Fe** | **K** | **Mg** | **Mn** | **Na** | **Zn** |
| Thoiras | (1) | 26/06/2020 | Leaves | 0.74 ±0.99 | 13.57 ± 1.01 | 0.54 ± 2.77 | **20.13** ± 1.82 | 6.21 ± 0.09 | 0.2 ± 0.70 | 0.21 ± 0.60 | 0.12 ± 2.43 |
| | (4) | 28/05/2021 | Leaves | 0.3* ± 2.21 | 10.44 ± 0.14 | 0.10* ± 15.23 | **23.37** ± 0.4 | 5.92 ± 1,43 | 0.12* ± 2.42 | 0.16* ± 2.12 | 0.26* ± 14.12 |
| | (5) | 25/06/2021 | Leaves | 0.16 ± 2.66 | 12.54 ± 0.22 | 0.09 ± 4.46 | **19.44** ± 0.83 | 6.82 ± 0.29 | 0.07 ± 1.07 | 0.12 ± 1.61 | 0.02* ± 22.63 |
| | (6) | 24/09/2021 | Leaves | 0.25 ± 2.72 | 16.86 ± 0.48 | 0.14 ± 3.79 | **9**.**99** ± 0.55 | 8.6 ± 0.45 | 0.12 ± 0.88 | 0.1 ± 1.22 | 0.01 * ± 51.94 |
| | (13) | 24/09/2021 | Stems | 0.15 ± 1.68 | 14.67 ± 0.69 | 0.14 ± 6.35 | **16.57** ± 0.70 | 5.21 ± 0.79 | 0.04* ± 0.80 | 0.41 ± 1.60 | 0.04* ± 218.1 |
| Saint Bauzille de Putois | (2) | 01/07/2020 | Leaves | 0.40 ± 0.32 | 8.88 ± 1.38 | 0.46 ± 1.71 | **16.95** ± 1.46 | 3.14 ± 1.11 | 0.01 ± 23.1 | 0.20 ± 3.10 | 0.17 ± 4.41 |
| | (3) | 29/04/2021 | Leaves | 0.36 ± 1.98 | 13.71 ± 0.49 | 0.28* ± 11.11 | **23.19** ± 0.79 | 3.28 ± 1.36 | 0.17* ± 0.68 | 0.18* ± 4.74 | 0.29* ± 6.26 |
| | (11) | 21/06/2021 | Leaves | 0.43 ± 1.92 | 10.33 ± 0.41 | 0.4 ± 6.24 | **21.02** ± 0.29 | 4.2 ± 0.12 | 0.04* ± 3.4 | 0.08* ± 2.76 | 0.67 ± 1.35 |
| | (12) | 16/07/2021 | Leaves | 0.45 ± 1.47 | 11.06 ± 0.44 | 0.51 ± 3.53 | **18.96** ± 0.57 | 3.88 ± 1.35 | 0.02* ± 3.92 | 0.08* ± 4.16 | 0.1 * ± 8.19 |
| Aulas | (7) | 21/06/2021 | Leaves | 0.14* ± 1.68 | 6.57 ± 1.06 | 0.08* ± 36.12 | **29.01** ± 0.66 | 3.71 ± 0.96 | 0.06* ± 0.45 | 0.12* ± 0.45 | 0.55* ± 4.33 |
| | (8) | 16/07/2021 | Leaves | 0.13* ± 2.29 | 7.1 ± 0.86 | 0.14* ± 10.16 | **20.8** ± 0.88 | 3.17 ± 1.25 | 0.03* ± 0.48 | 0.12* ± 3.87 | 0.06* ± 8.85 |
| Vigan | (9) | 24/06/2021 | Leaves | 0.1* ± 3.30 | 10.75 ± 0.34 | 0.14 ± 2.88 | **25.19** ± 0.59 | 5.87 ± 0.82 | 0.08* ± 1.37 | 0.22 ± 2.03 | 0.04* ± 10.95 |
| | (10) | 16/07/2021 | Leaves | 0.15* | 16.9 | 0.13* | **14.13** | 5.32 | 0.04* | 0.01* | 0.06* |
| | | | | ± 0.58 | ± 0.39 | ± 7.64 | ± 1.02 | ± 0.69 | ± 2.05 | ± 14.79 | ± 4.67 |

| | | | | | | | | | | | |
|---|---|---|---|---|---|---|---|---|---|---|---|
| *: values below the limit of quantification | | | | | | | | | | | |

**Table 2**

| Plant: *Salix alba* **(EcoKOx-Sa-(1))** | | | | | | | | | | | |
|---|---|---|---|---|---|---|---|---|---|---|---|
| **Harvesting site** | **N°** | **Harvesting date** | **Part of the plant** | **Al** | **Ca** | **Fe** | **K** | **Mg** | **Mn** | **Na** | **Zn** |
| Alsace | (1) | 01/08/2020 | Leaves | 0.08 ± 1.88 | 18.51 ± 0.41 | 0.14 ± 2.86 | 11.1 ± 0.36 | 1.85 ± 0.73 | 0.02* ± 1.21 | 0.06 ± 0.82 | 0.22 ± 1.81 |

| | | | | | | | | | | | |
|---|---|---|---|---|---|---|---|---|---|---|---|
| *: values below the limit of quantification | | | | | | | | | | | |

**Table 3**

| Plant: *Arundo donax* **(EcoKOx-Ad-(X)** X being from 1 to 8) | | | | | | | | | | | |
|---|---|---|---|---|---|---|---|---|---|---|---|
| **Harvesting site** | **N°** | **Harvesting date** | **Part of the plant** | **Al** | **Ca** | **Fe** | **K** | **Mg** | **Mn** | **Na** | **Zn** |
| Grabels | (1) | 07/07/2021 | stems | 0.01* ± 4.38 | 1.29 ± 0.74 | 0.02* ±14.96 | **31.25** ±0.11 | 0.85 ± 3.43 | 0.02* ± 1.1 | 0.08 ± 1.28 | 0.03* ±11.61 |
| | (2) | 01/10/2021 | stems | 0.01* ± 1.78 | 1.24 ± 0.76 | 0.02 ± 0.30 | **35.99** ± 0.52 | 0.71 ± 0.18 | 0.03 ± 0.36 | 0.10 ± 1.56 | 0.01* ± 8.02 |
| Mauguio | (3) | 09/11/2021 | stems | 0.02 ± 3.01 | 2.3 ± 0.98 | 0.04 ± 0.61 | **32.61** ± 1.34 | 1.66 ± 0.37 | 0.04 ± 0.37 | 0.19 ± 1.25 | 0.04 ± 1.43 |
| | (4) | 09/11/2021 | stems | 0.04 ± 0.43 | 2.48 ± 0.52 | 0.05 ± 0.66 | **33.13** ± 0.2 | 1.59 ± 0.17 | 0.04 ± 0.38 | 0.17 ± 0.82 | 0.05 ± 0.7 |
| | (5) | 09/11/2021 | stems | 0.03 ± 1.28 | 2.61 ± 0.27 | 0.05 ± 0.75 | **33.35** ± 0.31 | 2.01 ± 1.26 | 0.03 ± 0.97 | 0.19 ± 0.92 | 0.05 ± 0.79 |
| | (6) | 09/11/2021 | stems | 0.03 ± 3.21 | 2.86 ± 0.75 | 0.05 ± 0.46 | **32.58** ± 1.36 | 2.09 ± 0.84 | 0.03 ± 0.94 | 0.2 ± 0.81 | 0.05 ± 1.92 |
| Montferrier | (7) | 12/11/2021 | stems | 0* ± 4.47 | 2.74 ± 0 | 0* ± 62.24 | **35.19** ± 0.80 | 0* ± 53.82 | 0* ± 2.50 | 0.15 ± 0.61 | 0* ± 59.45 |
| | (8) | 12/11/2021 | stems | 0* ± 28.6 | 2.40 ± 3.20 | 0* ± 13.42 | **37.24** ± 1.01 | 0* ± 45.17 | 0* ± 2.71 | 0.07 ± 1.29 | 0* ± 15.18 |

| | | | | | | | | | | | |
|---|---|---|---|---|---|---|---|---|---|---|---|
| *: values below the limit of quantification | | | | | | | | | | | |

### Example 2: Preparation of the catalysts

The plant species is harvested and is subjected to a heat treatment at 550°C, under air flow, for 4 hours. The obtained residue can be directly used as a catalyst. This process is exactly the same than the one described in example 1 above. Thus, all the compositions described in example 1 above are compositions according to the invention and usable as catalysts.

**EcoKOx-Fj-(1)** are further submitted to the following additional treatments. **EcoKOx-Fj-(1)** is stirred in water during 3 hours at room temperature. The mixture is then filtrated on a sintered material of porosity 3. The solid residue is washed with 3x50 ml of water, and dried in an oven at 80°C. Then, it is subjected to controlled heat treatment in air (from 25 °C to 350 °C and to 550 °C for 4 h) giving **EcoKOx-Fj-(1)*** (7,12 g). On the other hand, the filtrate is evaporated in the open air and leads to **Vegetal KHCO₃ Fj-(1)** (7.1 g). Further heat treatment (from 25 °C to 200 °C and to 250 °C for 2 h) gives **Vegetal K₂CO₃ Fj-(1).**

Same processes were applied to **EcoKOx-Fj-(2),** to give **Vegetal KHCO₃ Fj-(2)** and **Vegetal K₂CO₃ Fj-(2),** and to **EcoKOx-Ad-(1),** to give **Vegetal KHCO₃ Ad-(1)** and **Vegetal K₂CO₃ Ad-(1).**

The metallic compositions of some of these materials were analyses by MP-AES and are given below.

**Table 4**

| | **Al** | **Ca** | **Fe** | **K** | **Mg** | **Mn** | **Na** | **Zn** |
|---|---|---|---|---|---|---|---|---|
| **EcoKOx-Fj-(1)*** | 0.39 ± 1.32 | 19.14 ± 0.52 | 0.52 ± 2.19 | **9.01** ± 0.79 | 5.71 ± 0.69 | 0.06* ± 1.32 | 0* ± 3.04 | 0.09* ± 22.45 |
| **Vegetal KHCO₃ Fj-(1)** | 0* ± 161.5 | 0.59* ± 0.92 | 0.12* ± 53.94 | 4**6.43** ± 0.50 | 0.24* ± 0.35 | 0* ± 297.8 | 0* ± 0.48 | 0* ± 79.26 |
| **Vegetal K₂CO₃ Fj-(1)** | 0* ± 20.22 | 1.62* ± 0.67 | 0.16* ± 35.77 | **55.06** ± 0.67 | 0.02* ± 14.52 | 0.01* ± 74.64 | 0* ± 0.22 | 0* ± 81.94 |
| **Vegetal K₂CO₃ Ad-(1)** | 0* ± 54.8 | 0.11* ± 1.69 | 0* ± 15.9 | **35.63** ± 0.47 | 0.04* ± 0.51 | 0* ± 62.2 | 0.21 ± 0.62 | 0.02* ± 14.0 |

| | | | | | | | | |
|---|---|---|---|---|---|---|---|---|
| *: values below the limit of quantification | | | | | | | | |

### Example 3: Structural characterization of the compositions

**EcoKOx-Fj-(2), Vegetal KHCO₃ Fj-(2)** and **Vegetal K₂CO₃ Fj-(2)** were analyzed by XRD, in order to detect the different species comprised in these compositions.

X-ray diffraction (XRD) data measurements were performed by using a BRUKER diffractometer (D8 advance, with a Cu Kα radiation λ=1.54086 Å) equipped with a Lynxeyes detector. Diffraction patterns were analysed with DIFFRAC-EVA software and several XRD database (Crystallography Open Data Base 2016, and PDF 2011, 2017 or 2018)

The results are given in the table below.

**Table 5**

| **Composition** | **Species** | **Formula** |
|---|---|---|
| **EcoKOx-Fj-(2)** | Sylvite | **KCI** |
| | Arcanite, sodium sulfate | **K₂SO₄,** Na₂SO₄ |
| | Fairchildite | K₂Ca(COₛ)₂ |
| | Quartz, calcium magnesium silicate, calcium silicate | SiO₂, CaMgSi₂O₆ |
| | Calcium carbonate | CaCO₃ |
| | Kalicinite | **KHCO₃** |
| | Potassium carbonate hydrate | **K₂CO₃. 1,5 H₂O** |
| | Hydrotalcite | (Mg₆Al₂(OH)₁₆(CO₃)(H₂O)_{4,5})_{0,25} |
| | Natrite | Na₂CO₃ |
| **Vegetal KHCO₃ Fj-(2)** | Sylvite | **KCI** |
| | Potassium carbonate hydrate | **K₂CO₃. 1,5 H₂O** |
| | Kalicinite | **KHCO₃** |
| | Hydrotalcite | (Mg₆Al₂(OH)₁₆(CO₃)(H₂O)_{4,5})_{0,25} |
| | Natrite | Na₂CO₃ |
| **Vegetal K₂CO₃ Fj-(2)** | Sylvite | KCI |
| | Arcanite | **K₂SO₄** |
| | Potassium carbonate hydrate | **K₂CO₃ . 1,5 H₂O** |
| | | K₄(CO₃)₂(H₂O)₃ |
| | Calcite - alliage Mg | (Ca,Mg)CO₃ |
| **EcoKOx-Ad-(1)** | Sylvite | **KCI** |
| | Arcanite, syn | **K₂SO₄** |
| | Potassium hydrogenocarbonate hydrate | **K₂H(CO₃)_{1,5}(H₂O)_{0,75}** |
| | Potassium hypomanganate | K₃(MnO₄) |
| | Manganese sulfide | MnS |
| Vegetal K₂CO₃ Ad-(1) | Sylvite | **KCI** |
| | Arcanite, syn | **K₂SO₄** |
| | Potassium carbonate hydrate | **K₂CO₃. 1,5 H₂O** |

Additional crystalline salts of **EcoKOx-Fj-(2)** were detected by EDS-EBSD, which is the combination of chemical data from energy dispersive X-ray spectrometry (EDS) and crystallographic data from Electron Backscatter Diffraction (EBSD).

**Table 6**

| | Species | Formula |
|---|---|---|
| **EcoKOx-Fj-(2)** | Oxyapatite | (CaPO₄)₆ CaO |
| | K-feldspath | K(Al,Si)₄O₈ |
| | Calcite | CaCO₃ |
| | Dolomite | CaMg(CO₃)₂ |

Each catalyst presents a polymetallic structure. Whatever the method of characterization, it appears that these compositions present atypical basic salts, most of which are almost unknown in organic synthesis.

### Example 4: Nucleophilic addition of Michael type

Equipments:
For the mecanosyntheses: Planetary Ball Mill Machine referenced TMAX-XQM-12 or RETSCH PM100.

For the syntheses under microwaves:
1) Anton Paar reactor = Anton Paar Multiwave Go^{™} is a microwave digestion system. A single magnetron delivers 850 W microwave power with a magnetron frequency at 2455 W. The maximal temperature is up to 250 °C. The vessel temperature is continuously monitored with an IR temperature sensor. A rotor 12HVT50 (rotor speed > 5 rpm) is placed with 12 high-performance pressure activated venting vessels (50 mL) made of PTFE-TFM HVT50 (Vₘᵢₙ = 3 mL, Vₘₐₓ = 25 mL, Pₘₐₓ = 20 bar).
2) CEM Discover 2.0 microwave = Microwave Accelerated Reaction System for Synthesis, single-mode microwave cavity, 240 V, 50/60 Hz, 5 A - 300 W - Magnetic stirring (variable speed) - Widest pressurized Pyrex^{®} vessel sizes (10 mL, Vmin = 0.2 mL, Vmax = 7 mL, Pmax = 30 bar, Tmax = 300 °C ; 35 mL, Vmin = 2.0 mL, Vmax = 25 mL, Pmax = 20 bar, Tmax = 300 °C ; 100 mL, Vmin = 15 mL, Vmax = 70 mL, Pmax = 17 bar, Tmax = 250 °C) - iWave^{®} temperature sensor, Activent^{®} Technology for releasing unwanted gaseous byproducts from the reaction.
3) MARS 6, 240V/50Hz, Characteristics: Mars 6 Synthesis, Multimode platform 1800 W - Magnetic stirring, pressures up to 55 bars, variable speed - Optical fiber temperature control - Cavity passage for cooling fluid on flask from 250mL to 5L - Possibility to work with carousels.

### Example 4.1

The addition of various Michael donors on various Michael acceptors was tested under several conditions, gathered in the following table.

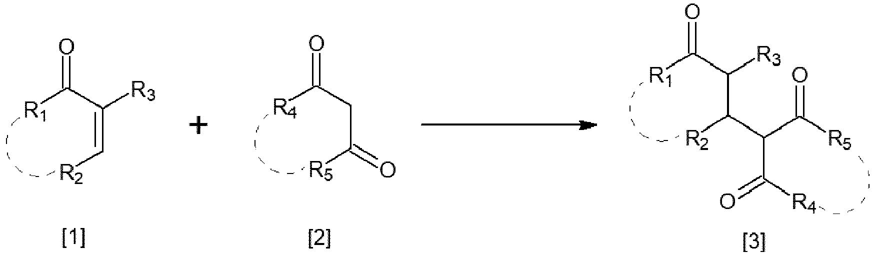

**Table 7**

| **Entry** | **R₁** | **R₂** | **R₃** | **R₄** | **R₅** | **Composition** | **K equiv** | **[1] equiv** | **[2] equiv** | **React. time** | **Conv %** | **Yield %** |
|---|---|---|---|---|---|---|---|---|---|---|---|---|
| 1 | Cyclopentyle | | H | OMe | OMe | EcoKOx-Sa-(1) | 0.2 | 1 | 1 | 2 h | 100 | 96^{(a)} |
| 2 | Cyclopentyle | | H | OMe | OMe | EcoKOx-Fj-(1) | 1.6 | 1 | 1 | 2 h | 100 | 96^{(a)} |
| 3 | Cyclopentyle | | H | OMe | OMe | EcoKOx-Fj-(2) | 0.8 | 1 | 1 | 2 h | 100 | 98^{(a)} |
| 4 | Cyclopentyle | | H | OMe | OMe | EcoKOx-Fj-(2) | 0.8 | 1 | 1 | 2 h | 100 | 97^{(b)} |
| 5 | Ph | Ph | H | OMe | OMe | EcoKOx-Fj-(2) | 0.8 | 1 | 1 | 5 h | 76 | 61^{(b)} |
| 6 | Cyclopentyle | | H | 6-member ring | | EcoKOx-Fj-(2) | 0.8 | 1 | 1 | 2 h | 75 | - |
| 7 | Cyclopentyle | | H | -Me | -Me | EcoKOx-Fj-(2) | 0.8 | 1 | 1 | 2 h | 24 | - |
| 8 | Cyclopentyle | | C₅H₁₁ | OMe | OMe | EcoKOx-Sa-(1) | 1 | 1 | 10 | 16 h | 67 (92/8) | - |
| 9 | Cyclopentyle | | C₅H₁₁ | OMe | OMe | EcoKOx-Fj-(2) | 2 | 1 | 10 | 6 h | 61 (92/8) | - |
| 10 | Cyclopentyle | | C₅H₁₁ | OMe | OMe | EcoKOx-Ad-(1) | 2 | 1 | 10 | 6 h | 66 (89/11) | - |
| 11 | Cyclopentyle | | C₅H₁₁ | OMe | OMe | Vegetal KHCO₃ **Fj-(2)** | 2 | 1 | 10 | 4 h | 40 (80/20) | - |
| 12 | Cyclopentyle | | C₅H₁₁ | OMe | OMe | Vegetal KHCO₃ **Fj-(2)** | 2 | 1 | 10 | 6 h | 91 (90/10) | - |
| 13* | Cyclopentyle | | C₅H₁₁ | OMe | OMe | Commercial KHCO₃ | 2 | 1 | 10 | 2 h | 0 | - |
| 14 | Cyclopentyle | | C₅H₁₁ | OMe | OMe | Vegetal K₂CO₃ Fj-(2) | 2 | 1 | 10 | 2 h | 64 (85/15) | - |
| 15 | Cyclopentyle | | C₅H₁₁ | OMe | OMe | Vegetal K₂CO₃ Fj-(2) | 2 | 1 | 10 | 4 h | 91 (90/10) | - |
| 16* | Cyclopentyle | | C₅H₁₁ | OMe | OMe | Commercial K₂CO₃ anhvdre | 2 | 1 | 10 | 3 h | 94 (90/10) | - |

| | | | | | | | | | | | | |
|---|---|---|---|---|---|---|---|---|---|---|---|---|
| *: comparative examples (a) Quantified GC yield (b) Isolated yield | | | | | | | | | | | | |

Protocol: In a 25 mL bowl containing 1/3 of 5 mm diameter stainless steel beads, molecule [2] (1 equiv. to 10 equiv. depending on R₃) and molecule [1] (1 equiv.) are added to the catalyst. The reaction mixture is ground for a given time (see table 7) with a rotation speed of 500 rpm (8.33 Hz) with a pause interval of 10 min every hour. The reaction medium is dissolved in ethyl acetate and filtered through cellulose. After a distillation to recover the excess molecule [2], the crude reaction can optionally be directly engaged in a decarboxylation reaction.

### Example 4.2: Recycling of the catalyst

The capacity to recycle **EcoKOx-Fj-(2)** was assessed. Once filtered, the catalyst is heat treated under air (25° then 350°C then 550°C for 4 h). In a 25 mL bowl containing 1/3 of 5 mm diameter beads, molecule [2] with R₄ = R₅ = OMe (1 equiv.) and molecule [1] with R₁ and R₂ linked to form cyclopentyl, R₃ = H (1 equiv.) are added to recycled **EcoKOx-Fj-**(2) (0.8 K-equiv.). The reaction mixture is ground for 2h with a rotational speed of 500 rpm (8.33 Hz) with a 10 min pause interval every hour. The reaction medium is dissolved in ethyl acetate and filtered through cellulose. The following results were obtained.

**Table 8**

| No. recycling | Conversion (%) | Yield (GC) (%) |
|---|---|---|
| 1 | 100 | 96 |
| 2 | 100 | 93 |
| 3 | 100 | 91 |
| 4 | 100 | 98 |

The catalyst performances are the same, even after 4 recycling.

### Example 4.3: Sequential nucleophilic addition reaction of Michael type/aldolic condensationldecarboxylation

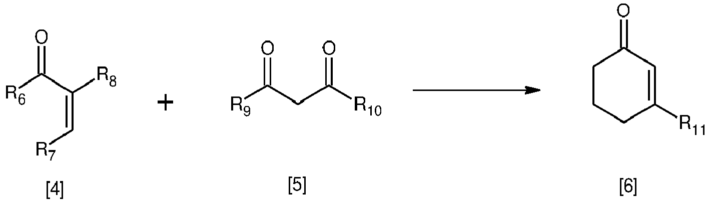

**Table 9**

| **Entry** | **R₆** | **R₇** | **R₈** | **R₉** | **R₁₀** | **R₁₁** | **Catalyst** | **K equiv** | **[4] equiv** | **[5] equiv** | **Protocol** | **Temperature** | **React. Time** | **Conv %** |
|---|---|---|---|---|---|---|---|---|---|---|---|---|---|---|
| 39 | Me | H | H | Me | OMe | Me | EcoKOx-Sa-(1) | 0.05 | 1 | 1 | 3 | 120 °C | 45 min | 96^{(b)} |
| 40 | H | H | H | Me | OMe | H | EcoKOx-Fj-(2) | 0.03 | 1 | 1 | 4 | 150 °C | 2 h 30 | 82^{(b)} |

| | | | | | | | | | | | | | | |
|---|---|---|---|---|---|---|---|---|---|---|---|---|---|---|
| (b) Isolated yield | | | | | | | | | | | | | | |

*Protocol 3:* In a 50 mL Teflon reactor, catalyst (486 mg, 0.14 mmol K, 0.05 equiv. K), the molecule [5] with R9 = -Me and R10 = -OMe (3.2 mL, 30 mmol, 1 equiv.), the molecule [4] with R6 = -Me, R7 = R8 = -H (2.5 mL, 30 mmol, 1 equiv.) and glycerol (4.5 mL, 60 mmol, 2 equiv.) are added. The Teflon reactor is then placed in the Anton Paar microwave reactor, whose method starts with a 5 min temperature rise to reach a plateau at 120 °C for 45 min in single reactor mode. The organics are extracted with ethyl acetate (3 x 20 mL) and the glycerol is washed with water (20 mL). The final product is distilled under vacuum (Eb = 42 °C at P = 0.0014 mbar, 3.17 g, 28.8 mmol, Rdt = 96%)

*Protocol 4:* In a 50 mL Teflon reactor, catalyst (237 mg, 1.03 mmol K-equiv., 0.03 K-equiv.), the molecule [5] with R9 = Me and R10 = OMe (3.4 mL, 30 mmol, 1 equiv.), the molecule [4] with R6 = R7 = R8 = H (2 mL, 30 mmol, 1 equiv.) and glycerol (4.5 mL, 60 mmol, 2 equiv.) are added. The Teflon reactor is then placed in the Anton Paar microwave reactor, the method of which starts with a 10 min temperature rise to reach a plateau at 150 °C for 2 h 30 min in single reactor mode. The organics are extracted with ethyl acetate (3 x 20 mL) and the glycerol is washed with water (20 mL). The final product is distilled under vacuum (2.36 g, Eb 1.4mbar= 20-22 °C, 24.6 mmol, Rdt = 82%).

Since acrolein and ethyl acetoacetate are available in natural form, the use of the composition according to the invention as a biobased catalyst leads to a 100% biobased cyclohexenone.

### Example 5: Aldolization/crotonisation and similar reactions

### Example 5.1

The cross aldol/cetol condensation between several reagents were performed. The results are gathered in the table below.

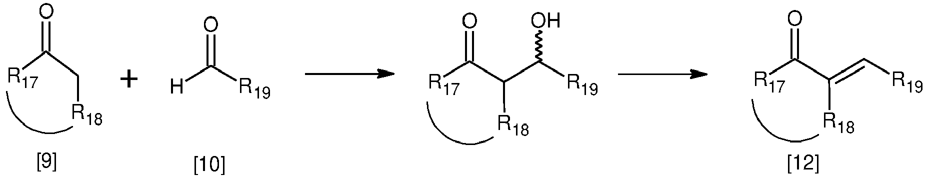

**Table 10**

| **Entry** | **R₁₇** | **R₁₈** | **R₁₉** | **Catalyst** | **K equiv** | **[9] equiv** | **[10] equiv** | **Protocol** | **Temperature** | **React. time** | **Conv %** | **Yield %** |
|---|---|---|---|---|---|---|---|---|---|---|---|---|
| 41 | cyclopentyle | | C₅H₁₁ | EcoKOx-Sa-(1) | 0.2 | 5 | 1 | 6 | 120 °C | 1 h 30 | 64 | 52 |
| 42 | cyclopentyle | | C₅H₁₁ | EcoKOx-Fj-(13) | 0.2 | 5 | 1 | 6 | 120 °C | 1 h 30 | 71% | - |
| 43 | cyclopentyle | | C₅H₁₁ | EcoKOx-Fj-(2) | 0.2 | 5 | 1 | 6 | 120 °C | 1 h 30 | 80 | - |
| 44 | cyclopentyle | C₅H₁₁ | EcoKOx-Fj-(1)* | 0.2 | 5 | 1 | 6 | 120 °C | 1 h 30 | 87 | - | |
| 45 | cyclopentyle | C₅H₁₁ | EcoKOx-Fj-(2) | 0.2 | 5 | 1 | 7 | 120 °C (5min) → 150 °C (25 min) | 30 min | 100 | 90 | |
| 46 | cyclopentyle | C₅H₁₁ | EcoKOx-Sa-(1) | 0.2 | 5 | 1 | 7 | 120 °C (5min) → 150 °C (25 min) | 30 min | 100 | 56 | |
| 47 | cyclopentyle | C₅H₁₁ | Commercial K₂CO₃ | - | 5 | 1 | 8 | 120 °C (5min) → 150 °C (25 min) | 30 min | 100 | - | |

*Protocol* 6: The reaction is divided into twelve 50 mL Teflon reactors. To each of the reactors, catalyst, cyclopentanone [9] and valeraldehyde [10] are added. The Teflon reactors are then placed in the Anton Paar microwave reactor (without stirring), whose method starts with a 5 min temperature rise to reach a plateau at 120 °C for 1 h 30 in multi-reactor mode. The reaction mixtures are pooled and filtered over sinter (pore size 4) using ethyl acetate (100 mL) which is evaporated under vacuum at 400 mbar with a bath temperature of 25 °C (Buchi B-100, Vaccuumbrand pump). A first distillation is performed to isolate excess [9] (cyclopentanone) (61 mL, E_{b} 98mbar= 58-61 °C, 690 mmol, 76% recovered). A second distillation is performed to isolate 2-pentylidene-cyclopentanone [12] (17.8 g, E_{b} 1.3mbar= 65 °C, 118 mmol, Rdt = 52%).

*Protocol 7:* In a 100 mL microwave reactor, catalyst, cyclopentanone [9] (56 mL, 0.633 mol, 5 equiv) and valeraldehyde [10] (14 mL, 0.132 mol, 1 equiv) are added. The reaction mixture is subjected to the CEM Discover 2.0 microwave reactor for 5 min at 120 °C then 25 min at 150 °C (Speed = slow). The operation is repeated 9 times. The reaction mixture is filtered under vacuum and washed with ethyl acetate (250 mL). The solvent is evaporated under vacuum. (2Z)-2-pentylidenecyclopentanone [12] is obtained after vacuum distillation in 56% yield (101 g, 663 mmol).

*Protocol 8:* In a 100 mL microwave reactor, K₂CO₃ (450 mg, 3.26 mmol, 0.2 equiv.), cyclopentanone [9] (8.7 mL, 94.12 mmol, 5 equiv.) and valeraldehyde [10] (2 mL, 18.8 mmol, 1 equiv.) are added. The reaction mixture is subjected to CEM Discover 2.0 microwave for 5 min at 120 °C and then 25 min at 150 °C (Speed = high).

### Example 5.2: Knoevenagel-type reaction

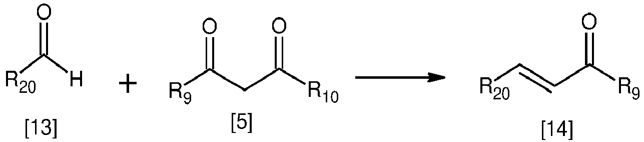

**Table 11**

| **Entry** | **R₂₀** | **R₉** | **R₁₀** | **Catalyst** | **K equiv** | **[13] equiv** | **[5] equiv** | **Temperature** | **React. Time** | **Conv %** |
|---|---|---|---|---|---|---|---|---|---|---|
| 48 | Ph | Me | OMe | EcoKOx-Fj-(2) | 0.07 | 1 | 1 | 150 °C | 2 h 30 | 28 |

Protocol: In a 50 mL Teflon reactor, catalyst (237 mg, 2.1 mmol K, 0.07 equiv.), methylacetoacetate (3.4 mL, 30 mmol, 1 equiv.), benzaldehyde (3 mL, 30 mmol, 1 equiv.), and glycerol (4.5 mL, 60 mmol, 2 equiv.) were added. The Teflon reactor is then placed in the Anto Paar microwave, the method of which starts with a 10 min temperature rise to reach a plateau at 150 °C for 2.5 hours in single reactor mode. Benzaldehyde is converted by about 28% to 4-phenyl-3-buten-2-one.

### Example 5.3: Dieckmann reaction

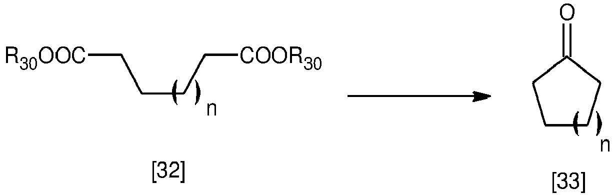

**Table 12**

| **Entry** | **R₃₀** | **n** | **Catalyst** | **K equiv** | **[32] equiv** | **Temperature** | **React. Time** | **Conv %** |
|---|---|---|---|---|---|---|---|---|
| 49 | Me | 1 | EcoKOx-Fj-(2) | 1 | 1 | 250 °C | 1 h | >99 |

Protocol: In a 10 mL Teflon reactor, catalyst (14 mg, 0.12 mmol, 0.05 equiv.), dimethyl adipate (410 µL, 2.5 mmol, 1 equiv.) and glycerol (205 µL, 2.5 mmol, 1 equiv.) were added. The Teflon reactor is then placed in the Anton Paar microwave at 250 °C, for 1 hour. The conversion was quantitative. No side product was detected.

### Example 6: Transfunctionalisation reactions

### Example 6. 1: Transesterification reactions

Transesterifications of fatty esters were performed as shown below.

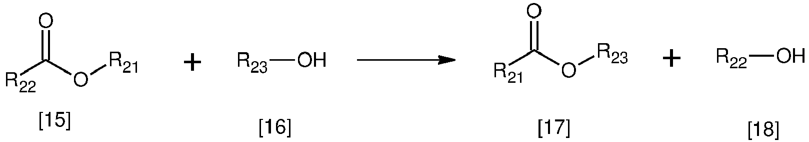

**Table 13**

| **Entry** | **R₂₁** | **R₂₂** | **R₂₃** | **Catalyst** | **K equiv** | **[15] equiv** | **[16] equiv** | **Temperature** | **React. Time** | **Conv %** |
|---|---|---|---|---|---|---|---|---|---|---|
| 50 | geranyl | H | Me | EcoKOx-Fj-(2) | 0.07 | 1 | 1 | 60 °C | 3 h | 98 |

Protocol: In a 10 mL flask equipped with a distillation system, under nitrogen, catalyst (116 mg, 1.05 mmol in K, 0.07 equiv. in K), geranyl formate (3 mL, 15 mmol, 1 equiv.), and anhydrous methanol (6 mL, 150 mmol, 10 equiv.) were introduced. The reaction was heated to 60 °C for 3 h. The methyl formate formed is distilled off as the reaction proceeds (533 mg, 8.9 mmol, 59% recovered). At the end of the reaction, the reaction crude is filtered. Evaporation of the methanol under vaccum yields geraniol (2.28 g, 14.8 mmol, Rdt = 98%).

Transesterifications of vegetable oils were also performed as shown below.

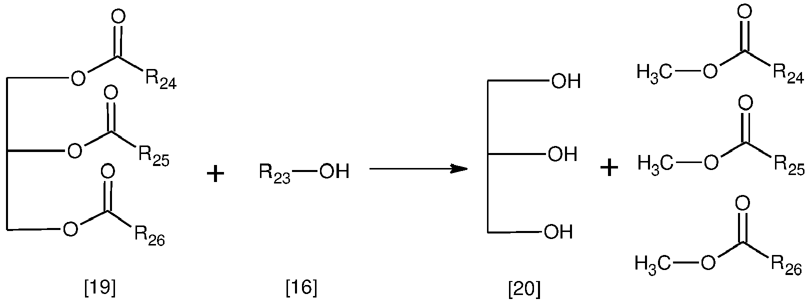

**Table 14**

| **Entry** | **R₂₃** | **Oil ([19])** | **Catalyst** | **K equiv** | **Protocol** | **Temperature** | **React. time** | **Conv %** |
|---|---|---|---|---|---|---|---|---|
| 51 | Et | Olive oil | EcoKOx-Fj-(2) | 1 | 11 | 120 °C | 30 min | 100 |
| 52 | Et | Grapeseed oil | EcoKOx-Fj-(2) | 1 | 11 | 120 °C | 30 min | 100 |
| 53 | Et | Coconut oil | EcoKOx-Fj-(2) | 1 | 11 | 120 °C | 30 min | 100 |
| 54 | Et | Sunflower oil | EcoKOx-Fj-(2) | 1 | 11 | 120 °C | 30 min | 100 |
| 55 | Et | Rapeseed oil | EcoKOx-Fj-(2) | 1 | 11 | 120 °C | 30 min | 100 |
| 56 | Et | Olive oil | EcoKOx-Ad-(1) | 0.05 | 12 | 120 °C | 30 min | 100 |
| 57 | Et | Grapeseed oil | EcoKOx-Ad-(1) | 0.05 | 12 | 120 °C | 30 min | 100 |
| 58 | Et | Coconut oil | EcoKOx-Ad-(1) | 0.05 | 12 | 120 °C | 30 min | 100 |
| 59 | Et | Sunflower oil | EcoKOx-Ad-(1) | 0.05 | 12 | 120 °C | 30 min | 100 |
| 60 | Et | Rapeseed oil | EcoKOx-Ad-(1) | 0.05 | 12 | 120 °C | 30 min | 100 |
| 61 | Et | Sunflower oil | EcoKOx-Ad-(2) | 0.05 | 12 | 120 °C | 30 min | 90 |

### All yields are based on HPLC-UV data with reference to standard compounds.

If ethanol is replaced by methanol, the reaction is complete after 15 min instead of 5h in the classical batch mode. With 0.5 equiv. of catalyst, the reaction leads to a monotransesterification after 30 min, which allows the preparation of a diglyceride.

*Protocol 11*: In a 35 mL microwave reactor equipped with a magnet bar, catalyst (682 mg, 3 mmol K, 1 equiv. K), oil (3 mmol, 1 equiv.), and ethanol (2 mL, 34 mmol, 11 equiv.) are introduced. The reaction mixture is pre-agitated for 2 min and then introduced in a microwave activation CEM Discover 2.0 reactor (with agitation) with a 2 min temperature ramp to 100 °C for 30 min.

*Protocol 12:* In a 35 mL microwave reactor equipped with a magnet bar, catalyst (22 mg, 0.15 mmol K, 0.05 equiv), oil (3 mmol, 1 equiv), and ethanol (2 mL, 34 mmol, 11 equiv) are introduced. The reaction mixture is pre-agitated for 2 min and then introduced in a microwave activation CEM Discover 2.0 reactor (with agitation) with a 2 min temperature ramp to 100 °C for 30 min.

### Example 6.2: Transcarbonatation reactions

Transcarbonatation reactions were performed as shown below.

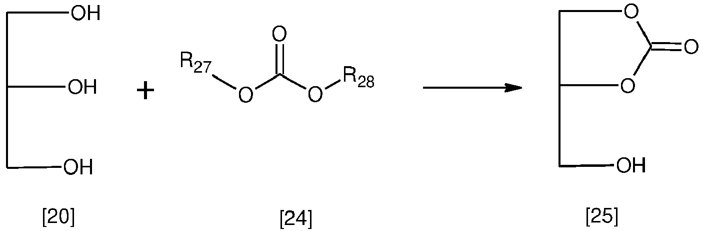

**Table 15**

| **Entry** | **R₂₇** | **R₂₈** | **Catalyst** | **K equiv** | **[20] equiv** | **[24] equiv** | **Temperature** | **React. time** | **Conv %** |
|---|---|---|---|---|---|---|---|---|---|
| 41 | Et | Et | EcoKOx-Ad-(1) | 0.05 | 1 | 1 | 150 | 45 min | 85 |

Protocol: In a 10 mL microwave reactor equipped with a magnet bar, catalyst (70.8 mg, 0.543 mmol K, 0.05 equiv. K), glycerol (1 g, 10.86 mmol, 1 equiv.), and diethylcarbonate (1.3 mL, 10.86 mmol, 1 equiv.) are introduced. The reaction mixture is pre-stirred for 2 min and then introduced into the CEM Discover 2.0 microwave reactor with a 2 min temperature ramp to 150 °C for 45 min. The conversion of glycerol to 4-hydroxymethyl-1,3-dioxolan-2-one is evaluated by HPLC showing 85% conversion. The reaction mixture is diluted in ethyl acetate and washed with water. The organic phase is recovered, dried with anhydrous MgSO4. The solvent is removed by rotary evaporator and the reaction mixture is dried under vacuum.

### Example 7: Oxydative hydroxylation reactions

Oxydative hydroxylation of para-hydroxybenzaldehyde was performed as shown below.

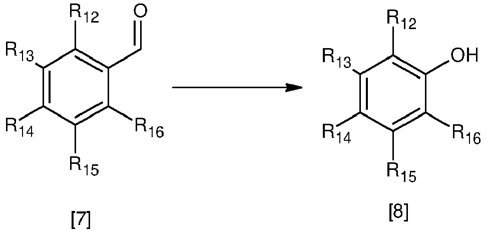

**Table 16**

| **Entry** | **R₁₂** | **R₁₃** | **R₁₄** | **R₁₅** | **R₁₆** | **Catalyst** | **K equiv** | **[7] equiv** | **Temperature** | **React. time** | **Conv %** |
|---|---|---|---|---|---|---|---|---|---|---|---|
| 62 | H | H | OH | H | OH | EcoKOx-Fj-(2) | 0.07 | 1 | r.t. | 3h | 82 |

Protocol: In a 10 mL flask, the molecule [7] with R12 = R13 = R15 = R16 = -H and R14 = -OH (1.22 g, 10 mmol, 1 equiv.), 40% w/v hydrogen peroxide (1.5 mL), catalyst (158 mg, 0.68 mmol K, 0.07 equiv. K) are added. The reaction mixture is stirred at room temperature for 3 h. The organic products are washed with water (3 x 20 mL) and extracted with ethyl acetate (3 x 20 mL). The organic phases are pooled and the ethyl acetate is evaporated under vacuum. Hydroquinone is obtained with 82% conversion.

### Example 8: Synthesis of methyl dihydrojasmonate

The synthesis of methyl dihydrojasmonate was performed by the following process:
a) reacting cyclopentanone with pentanal to obtain a compound [28], according to entries 42 to 46 of Table 10,
b) performing an isomerization of compound [28] to obtain compound [29],
c) reacting compound [29] with dimethylmalonate to obtain a compound [30], according to entries 8-12 or 14-15 of Table 7. After decarboxylation, [29] and [31] are easily separated by distillation. [29] can be engaged in another Michael addition.
d) performing a decarboxylation of compound [30], preferably under microwaves, to obtain methyl dihydrojasmonate.

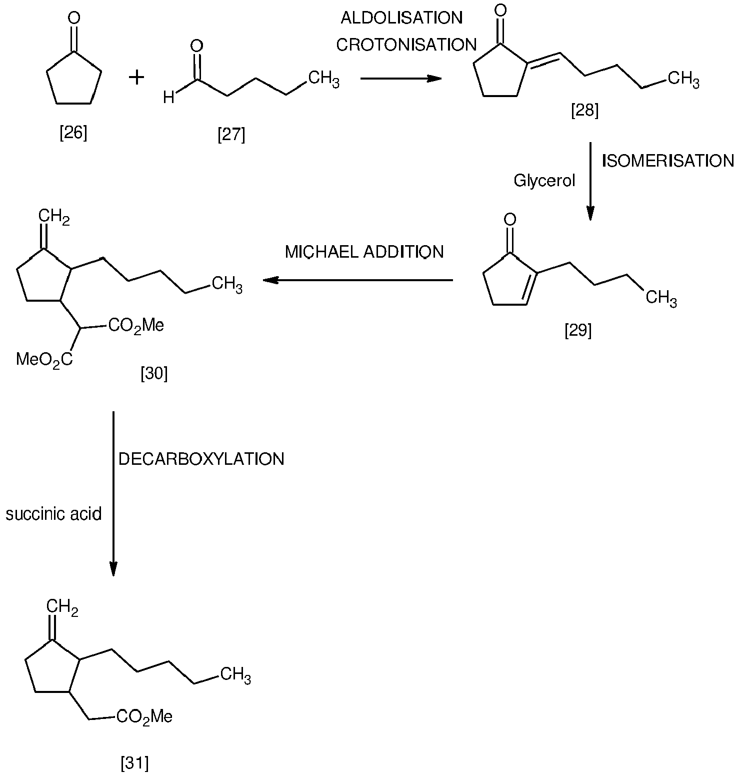

*Isomerization protocol (step b)):* The reaction is divided into 12 x 50 mL Teflon reactors. To each of the reactors, 2-pentylidenecylopentanone [29] (1 mL, 6.57 mmol, 1equiv.) and glycerol (5 mL, 67.9 mmol, 10 equiv.) are added. The Teflon reactors are then placed in an Anton Paar microwave, whose method starts with a 5 min temperature rise to reach a plateau at 150 °C for 2 h in multi-reactor mode. The reaction mixtures are combined for extraction with ethyl acetate (200 mL) and washing with water (200 mL). The organic phase is dried with anhydrous MgSO₄ and then evaporated under vacuum (Buchi B-100, Vacuumbrand pump). 2-Pentylcyclopent-2-en-1-one is obtained as a brown oil (11.4 g, Yield = 95%). The crude product [30] is directly used in the next step.

*Decarboxylation protocol (step d)):* In a 100 mL CEM Discover 2.0 microwave reactor equipped with a magnet bar, product [30] (10 g, 35.2 mmol, 1 equiv.) and succinic acid (8.30 g, 70.3 mmol, 2 equiv.) are added. The reaction mixture is microwaved with a 2 min ramp to 250 °C for 1 h. The product [31] is obtained after distillation (E_{b 7.10-2 mbar}= 95-96 °C).

Both aldolisation/crotonisation step and Michael addition step can be performed with all the compositions according to the invention, i.e. catalyst EcoKOx-Fj-(X), EcoKOx-Sa-(X), EcoKOx-Ad-(X), Vegetal KHCO₃ and Vegetal K₂CO₃.

## Claims

1. Composition comprising K₂CO₃, KCI, and optionally K₂SO₄ and/or KHCO₃, wherein the weight content of potassium is between 9.0 and 60.0 % relative to the total weight of the composition, preferably between 10.0 and 50.0%, more preferably between 10.0 and 40.0%, advantageously between 20.0 and 40.0%.

2. Composition according to claim 1, further comprising sodium, calcium and magnesium.

3. Composition according to claim 1 or 2, further comprising iron and aluminum.

4. Composition according to any of claims 1 to 3, being substantially devoid of calcium hydroxide Ca(OH)₂.

5. The composition according to any of claims 1 to 4, **characterized in that** it is substantially devoid of metals selected from the group of platinoids and in particular Pt, Pd or Rh, and/or of rare earths, in particular Ce, Eu, and Yb; and/or of metalloids, in particular B, Ge, As, Sb and Te.

6. The composition according to any of claims 1 to 5, **characterized in that** it is substantially devoid of transitions metals selected from the group consisting of Sc, Y, Ti, Zr, Hf, V, Nb, Ta, Cr, Mo, W, Tc, Re, Ru, Os, Co, Rh, Ir, Ni, Pd, Pt, Cu, Ag, Au ad Cd.

7. Method for the preparation of a composition according to any one of claims 1 to 6, comprising the following steps:
- selecting a plant, wherein the above-ground parts of said plant comprising between 10.0 and 40.0 % by weight of potassium,
- performing a heat treatment under air and at a temperature between 450 °C and 650°C of the above-ground parts of said plant, and
- obtaining the composition.

8. Method according to claim 7, **characterized in that** it is devoid of a step of acid treatment and/or is devoid of a basic treatment and/or is devoid of a purification step, for example by an ion exchange resin, a liquid-liquid extraction, a selective precipitation or a liquid/solid extraction and/or is devoid of an activation step.

9. Method according to claim 7 or 8, **characterized in that** the plant belongs to the genus Fallopia, Salix or Arundo.

10. Method of carrying out a chemical synthesis reaction comprising a step of contacting the composition according to any one of claims 1 to 6 with the reactant(s) of a chemical synthesis reaction, said chemical synthesis reaction being selected from the group consisting of 1,4-nucleophilic additions, aldolization/crotonisation reactions and similar reactions, transfunctionalisation reactions and oxidative hydroxylation reactions.

11. Method according to claim 10, **characterized in that** the step of contacting the composition with the reactant(s) of the 1,4-nucleophilic additions is carried out in the absence of organic solvent, preferably in the absence of any solvent, more preferably by mechanosynthesis or under microwaves, advantageously by mechanosynthesis.

12. Method according to claim 10 or 11, **characterized in that** the 1,4-nucleophilic additions are carried out in the presence of an amount of composition corresponding to between 0.01 equivalents and 5 equivalents of K relative to the limiting reactant, preferably between 0.05 equivalents and 4 equivalents, more preferably between 0.1 equivalent and 3 equivalents, advantageously between 0.1 equivalent and 2.5 equivalents.

13. Method according to any one of claims 10 to 12, **characterized in that** the chemical synthesis reaction comprises a 1,4-nucleophilic addition, followed by an aldolization/crotonisation reaction, followed by a decarboxylation reaction.

14. Method according to claim 10, **characterized in that** the aldolization/crotonisation reactions and similar reactions are carried out in the presence of an amount of composition corresponding to between 0.01 equivalents and 2.0 equivalents of K relative to the limiting reactant, preferably between 0.02 equivalents and 1.0 equivalents, more preferably between 0.05 equivalents and 0.5 equivalents.

15. Method according to claim 10, **characterized in that** the transfunctionalisation reactions are carried out in the presence of an amount of composition corresponding to between 0.01 equivalents and 5.0 equivalents of K relative to the limiting reactant, preferably between 0.02 equivalents and 2.5 equivalents, more preferably between 0.05 equivalents and 1.5 equivalents.

16. Method according to any one of claim 10, claim 14 and claim 15, **characterized in that** the aldolization/crotonisation reactions and similar reactions, and/or the transfunctionalisation reactions and/or the oxidative hydroxylation reactions are carried out under microwaves.

17. Method for the preparation of methyl dihydrojasmonate, comprising the following steps:
a) reacting cyclopentanone with pentanal in the presence of a composition according to any of claims 1 to 6, to obtain a compound of formula [28] 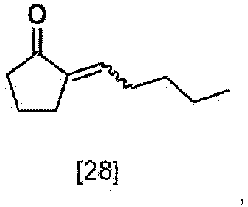
b) performing an isomerization of compound of formula [28], preferably under microwaves, to obtain compound of formula [29] 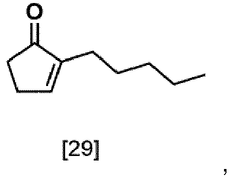
c) reacting compound of formula [29] with dimethyl malonate in the presence of a composition according to any of claims 1 to 6, to obtain a compound of formula [30] 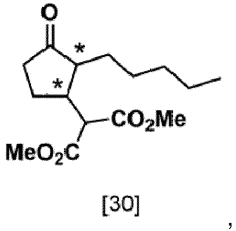
d) performing a decarboxylation of compound of formula [30], preferably under microwaves, to obtain methyl dihydrojasmonate.

18. Use of a composition according to any one of claims 1 to 6, as a catalyst of a chemical synthesis reactions.
